# EUROPEAN PATENT APPLICATION

(11) **EP 4 563 135 A1**
(43) Date of publication of application: **04.06.2025**
(21) Application number: 22953277.5
(22) Date of filing: 15.12.2022
(51) Int. Cl.: A61H 5/00, A61F 9/04, G02B 27/01, G02B 3/00

(54) **VISION TRAINING APPARATUS AND VISION TRAINING METHOD**

(30) Priority: 29.07.2022 KR 20220094834
(71) Applicant: Edenlux Corporation, Changwon-si, Gyeongsangnam-do 51542 (KR)
(72) Inventor: PARK, Sung Yong, Gyeonggi-do 47710 (KR)
(74) Representative: Samson & Partner Patentanwälte mbB
(86) International application number: PCT/KR2022/020444
(87) International publication number: WO 2024/025055

(57) **Abstract**

A vision training device according to an embodiment of the present invention includes a deviation angle determining unit that determines a deviation angle and a deviation direction of a user, a strabismus correction value determining unit that determines at least one or more correction prism diopters and a correction direction based on the deviation angle and the deviation direction, a vision training unit that determines at least one of the correction prism diopters as a training range and guides movement of the user's eye in the deviation direction and the correction direction, and a refraction angle adjusting unit that changes a refraction angle of light entering the user's eye according to the deviation angle or at least one or more correction prism diopters included in the training range. Through vision training, weak eye muscles, which are the root cause of strabismus, can be strengthened.

## Description

### TECHINICAL FIELD

The present invention relates to a vision training device and a vision training method. More particularly, the present invention relates to a vision training device and a vision training method for patients having strabismus or phoria.

### BACKGROUND ART

Strabismus refers to any misalignment of the eyes. Strabismus refers to a condition where, when looking at an object, one eye is focused on an object while the other eye is not. Phoria, also known as latent tropia, refers to a condition where both eyes are aligned properly under normal circumstances, but strabismus appears when one eye is blocked. Hereinafter, "strabismus" is used as a term representing phoria (latent tropia) as well as strabismus (or tropias).

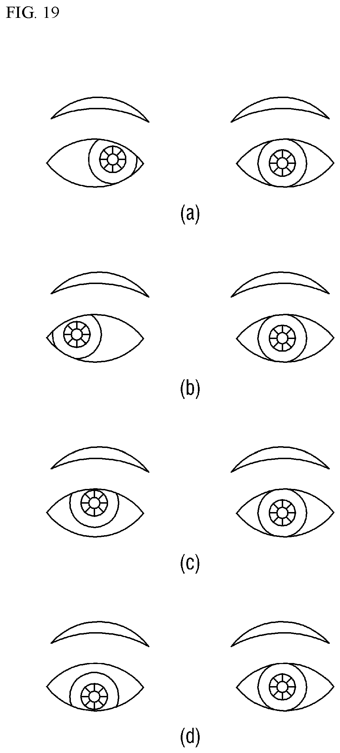

As shown in FIG. 19, strabismus is categorized into esotropia (a), exotropia (b), hypertropia (c), and hypotropia (d) depending on a deviation direction, and there is intermittent exotropia where one eye rotates outwards when the eyes experience fatigue from looking at one spot for a long time, while the eyes are normal under usual circumstances.

Strabismus can have various causes, including brain diseases such as brain tumors, metabolic disorders like hyperlipidemia, diabetes, and hypertension, thyroid abnormalities, inflammation of the eye muscles, or injuries around the eyes, and in many cases, the cause is not unknown, and strabismus can also be congenital.

Patients with strabismus experience symptoms such as blurred vision, double vision, headaches, reduced depth perception, and excessive squinting, and in some cases, strabismus can also lead to amblyopia (lazy eye). 80% of strabismus patients are children and adolescents.

A known treatment method for strabismus involves therapy conducted at vision training centers. However, this therapy method has drawbacks in terms of cost and time, as patients need to visit the vision training center over a long period.

Another known treatment method for strabismus is surgical intervention. However, surgical intervention is only possible for patients with a deviation angle of 15 prism diopters or more, and it is unsuitable for children and adolescents with an angles of deviation less than 15 prism diopters. Additionally, surgical intervention requires general anesthesia, which can pose risks of side effects for children and adolescents.

Additionally, surgical methods for strabismus have limitations as treatment options because two techniques are mainly used regardless of the type of strabismus: recession, where the eye muscles are lengthened and reattached further back, and resection, where the muscles are cut and reattached. Additionally, surgical methods for strabismus involve weakening the stronger muscles around the eye, leading to the overall weakening of the eye muscles after treatment.

Therefore, there is a need for non-invasive treatment intervention for strabismus that can effectively improve symptoms without side effects, even for children and adolescents.

### DISCLOSURE OF THE INVENTION

It is an object of the present invention to provide a vision training device and method, which are non-invasive and capable of effectively improving the symptoms of strabismus and vision without surgical intervention or procedures.

The problems to be solved by the present invention are not limited to those mentioned above, and it will be clearly understood by those skilled in the art from the following description that other problems not mentioned above are also included.

A vision training device according to an embodiment of the present invention preferably includes a storage unit that stores a deviation angle of a user and a control unit that guides movement of the user's eye based on the deviation angle.

Preferably, the control unit includes a strabismus correction value determining unit that determines a strabismus correction value based on the deviation angle and a vision training unit that sets a training range based on the strabismus correction value and guides the movement of the user's eye based on the training range.

Preferably, the storage unit stores information indicating a deviation direction, the strabismus correction value includes a correction direction opposite to the deviation direction and at least one or more correction prism diopters smaller than the deviation angle, and the training range includes at least one or more of the at least one or more correction prism diopters.

Preferably, the training range includes an interval where fusion is performed relatively quickly and an interval where fusion is performed relatively slowly.

A vision training device according to an embodiment of the present invention preferably includes a storage unit that stores information indicating deviation of the user's eye and at least one or more pieces of correction information for correcting the deviation of the user's eye and a control unit that guides movement of the user's eye in a direction different from a direction of the deviation based on at least one of the at least one or more pieces of correction information.

Preferably, the at least one or more pieces of correction information differ in time taken for fusion of both eyes.

Preferably, the at least one or more pieces of correction information guides the user's eye to move in a direction opposite to the direction of the deviation.

Preferably, the at least one or more pieces of correction information are at least one or more prism diopters.

Preferably, the at least one or more prism diopters includes at least one or more or a combination of a prism diopter associated with a deviation angle of the user's eye, a prism diopter which is smaller than the deviation angle and smallest to achieve fusion of the user's eye, and a prism diopter which is smaller than the deviation angle and larger than the smallest prism diopter.

Preferably, the prism diopter which is smaller than the deviation angle and larger than the smallest prism diopter is a prism diopter at which double vision becomes single vision.

A vision training method according to an embodiment of the present invention includes measuring a fusion adaptation time for each of a plurality of refraction angles smaller than a deviation angle of a user's eye, determining a training time for changing the refraction angle to the plurality of refraction angles based on the fusion adaptation time, determining at least one or more of the plurality of refraction angles as a training range, and changing the refraction angle to the at least one or more refraction angles included in the training range according to the training time to guide movement of the user's eye.

Preferably, the at least one or more refraction angles included in the training range include a refraction angle at which double vision becomes single vision.

Preferably, the at least one or more refraction angles included in the training range include the smallest refraction angle at which both eyes achieve fusion.

Preferably, the at least one or more refraction angles included in the training range include a deviation angle of the user's eye.

Preferably, the fusion adaptation times taken to achieve function of both eyes, which are measured for the plurality of refraction angles, are different from each other.

### BRIEF DESCRIPTION OF THE DRAWINGS

FIG. 1 is a block diagram illustrating a vision training device according to an embodiment of the present invention.
FIG. 2 is a perspective view illustrating a vision training device according to an embodiment of the present invention.
FIG. 3 is a perspective view illustrating a vision training device according to an embodiment of the present invention.
FIG. 4 is a diagram illustrating a sensor unit that captures a condition of the eye according to an embodiment of the present invention.
FIG. 5 is a perspective view illustrating a vision training device having a blocking unit.
FIG. 6 is a perspective view illustrating a prism lens assembly and a gear according to an embodiment of the present invention.
FIG. 7 is a cross-sectional view of a prism lens assembly taken along line A-A' of FIG. 5.
FIGS. 8(a) to 8(d) are schematic diagrams illustrating prism lenses, which are viewed from an X-Y plane according to an embodiment of the present invention.
FIGS. 9(a) to 9(d) are views illustrating the user's eye with exotropia and multiple prism lenses, which are viewed from an X-Y plane.
FIGS. 10(a) to 10(d) are views illustrating the user's eye with esotropia and multiple prism lenses, which are viewed from an X-Y plane.
FIGS. 11(a) to 11(d) are schematic diagrams illustrating prism lenses, which are viewed from a Y-Z plane according to an embodiment of the present invention.
FIGS. 12(a) to 12(d) are views illustrating the user's eye with hypertropia and multiple prism lenses, which are viewed from a Y-Z plane.
FIGS. 13(a) to 13(d) are views illustrating the user's eye with hypotropia and multiple prism lenses, which are viewed from a Y-Z plane.
FIG. 14 is a diagram for describing a vision training device according to an embodiment of the present invention.
FIG. 15 is a diagram for describing a vision training device with an augmented reality (AR) function according to another embodiment of the present invention.
FIG. 16 is a diagram describing a vision training device with a virtual reality (VR) function according to another embodiment of the present invention.
FIG. 17 is a flowchart showing a vision training method according to an embodiment of the present invention.
FIG. 18 is a view for describing a deviation direction and a correction direction.
FIGS. 19(a) to 19(d) are schematic diagrams illustrating various strabismus types.
FIG. 20 is a view for describing a vision training method according to an embodiment of the present invention.
FIG. 21 is a view for describing a vision training method according to an embodiment of the present invention.
FIG. 22 is a view for describing a vision training method according to an embodiment of the present invention.
FIG. 23 is a view for describing a vision training method according to an embodiment of the present invention.
FIG. 24 is a view for describing a vision training method according to an embodiment of the present invention.
FIG. 25 is a view for describing a vision training method according to an embodiment of the present invention.
FIG. 26 is a view for describing a vision training method according to an embodiment of the present invention.
FIG. 27 is a view for describing a vision training method according to an embodiment of the present invention.

### BEST MODE FOR CARRYING OUT THE INVENTION

Hereinafter, a vision training device and a vision training method according to an embodiment of the present invention will be described in detail.

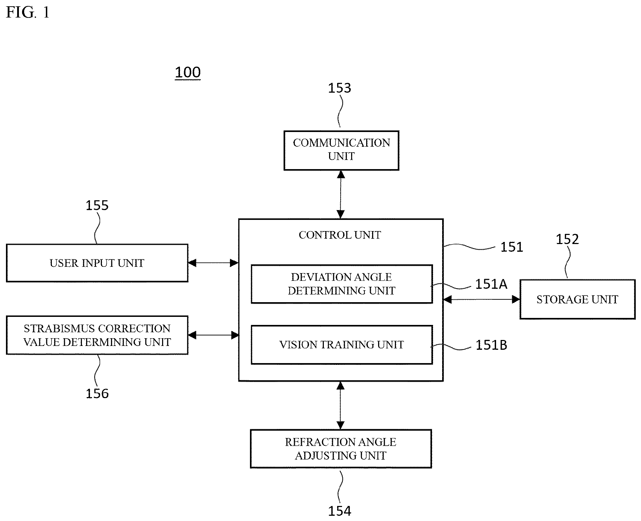

FIG. 1 is a block diagram illustrating a vision training device 100 according to an embodiment of the present invention.

The vision training device 100 according to the embodiment of the present invention includes a control unit 151, a storage unit 152, a communication unit 153, a refraction angle adjusting unit 154, a user input unit 155, and a deviation angle determining unit 156.

The deviation angle determining unit 156 determines a deviation angle and a deviation direction of the eye of the user having strabismus. Here, the "deviation angle" refers to the degree of strabismus that represents the amount of deviation of the eye when looking straight ahead and is generally indicated by a refraction angle such as a prism diopter (PD).

Here, the "deviation angle" is denoted by a single prism diopter and also includes a certain range of deviation amount centered around a certain prism diopter (hereinafter, "deviation angle range"). For example, when the "deviation angle" is denoted as a 14 prism diopter, it should be understood that the 14 prism diopter includes the deviation angle range from a 14.4 prism diopter to a 13. 6 prism diopter. In the present invention, an example in which the deviation angle is denoted by using the prism diopters (PD) is described, but the deviation angle according to the present invention is not limited to an example using the PD and can also be indicated by other units.

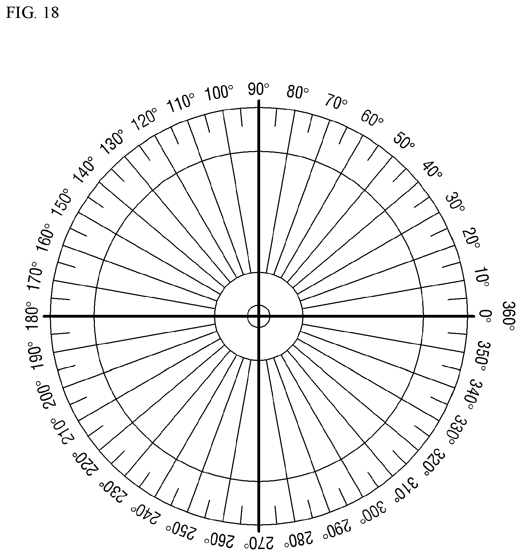

The deviation direction indicates the deviation direction of the eye when looking straight ahead and can be represented by using an angle as shown in FIG. 18.

The prism diopter (PD) is a value representing the degree to which light is refracted when passing through a prism lens and is denoted by △. One unit of prism diopter (△) (1 PD (△)) means a refractive deviation of 1 cm at a distance of 100 cm.

The deviation angle and the deviation direction can generally be determined by an ophthalmologist through tests such as the cover test, modified Thorington test (MTT), Maddox rod test (MRT), and alternate prism cover test (PCT). However, in the present invention, the deviation angle and the deviation direction are determined by the deviation angle determining unit 156. When an ophthalmologist determines the deviation angle and the deviation direction, the deviation angle and the deviation direction of the user determined by the ophthalmologist can be obtained and stored in the storage unit 152 through the user input unit 155 or the communication unit 152. [64] To determine the deviation angle, the deviation angle determining unit 156 measures a fusion adaptation time at each prism diopter while changing the prism diopter with intervals of predetermined units (for example, in units of 0.5 PDs or in units of 1 PDs), and detects fusion state information of the user's eye.
Here, the fusion adaptation time refers to a time taken for the user's eye to achieve fusion at a given prism diopter, and the fusion state information includes at least one or more or a combination of a slow fusion (SF) start point, a separation point, a recovery point, an SF interval, and a fast fusion (FF) interval. These fusion adaptation time and the fusion state information may change as the user's vision improves over time or through vision training, so it is desirable to measure or determine the fusion adaptation time and the fusion state information periodically (for example, monthly). The deviation angle determining unit 156 may measure the fusion adaptation time or determine the fusion state information in a corrected eye state, for example, in a state where prism glasses are worn.

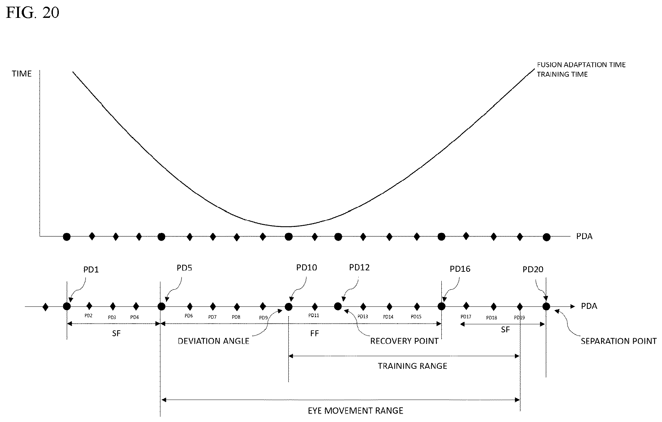

In FIG. 20, PDA represents an axis indicating the prism diopters, the left side of the axis represents prism diopters greater than the deviation angle of the user, while the right side represents prism diopters smaller than the deviation angle of the user. In other words, PD1 to PD9 represent prism diopters greater than the deviation angle (PD10), while PD11 to PD20 represent prism diopters smaller than the deviation angle.

The SF interval represents a prism diopter range where function and double vision alternately occur, so it takes a long time to achieve final function, leading to slow function, and is a range of PD1 to PD5 and a range of PD16 to just before PD20 in FIG. 20.

The FF interval represents a prism diopter range (deviation angle range), where fusion occurs quickly, which is a range of PD5 to PD16 in FIG. 20.

When the prism diopter is changed along the PDA from a value greater than the deviation angle of the user towards the deviation angle, in the prism diopter range (PD1 to PD5) (SF interval), the image is seen blurry as it gets closer to the deviation angle. In the prism diopter range corresponding to the deviation angle range (PD5 to PD16), fusion occurs quickly, and the image is seen clearly. Then, when the prism diopter is further changed from the deviation angle towards the 0 prism diopter, fusion occurs slowly again in the prism diopter range (PD16 to PD20) (SF interval). Then, when the prism diopter is changed further towards 0 prism diopter, at the prism diopter (PD20) (separation point), double vision occurs, and fusion no longer occurs. Then, when the prism diopter is changed from the separation point towards the deviation angle, the double vision becomes single vision at a specific prism diopter (PD12) (recovery point). The recovery point may appear at any prism diopter within the FF interval (PD12 in FIG. 20) or at a prism diopter corresponding to a start point to enter the FF interval (PD16 in FIG. 20) from the SF interval near the separation point. It is because the recovery point appears depending on the user' vision characteristics.

Here, the SF start point means the prism diopter (PD1 and PD16) where binocular fusion occurs but takes a relatively long time to achieve. The SF interval is a range in which fusion and double vision for a target occur alternately and repeatedly. The separation point of the eye refers to the prism diopter (PD20) where fusion no longer occurs, and the target appears as two images. At the separation point and below, fusion of the eyes no longer happens. The recovery point of the eye refers to the prism diopter (PD12) where the double vision becomes a single vision again.

As shown in FIG. 20, fusion occurs slowly in the SF interval and occurs quickly in the FF interval. Fusion does not occur from the prism diopter or smaller corresponding to the separation point. The fusion adaptation time is shortest at the deviation angle, meaning that fusion occurs fastest at the deviation angle. It is preferable for the deviation angle determining unit 156 to determine the prism diopter, at which fusion occurs fastest in the FF interval, as the deviation angle.

The deviation angle determining unit 156 stores the measured fusion adaptation time at each prism diopter in the storage unit 152, and the fusion adaptation time is used to determine a training time for each prism diopter. Intervals of the prism diopters for which the deviation angle determining unit 156 measures the fusion adaptation time are determined according to the user's vision characteristics and may be input through the user input unit 155 or fixed units such as 0.5 PD or 1 PD may be used. The deviation angle determining unit 156 preferably measures the fusion adaptation time periodically, for example, monthly, and determines the fusion state information and a training range because the training range may change due to vision improvement through vision training.

The deviation angle determining unit 156 may include a sensor which is capable of measuring or determining at least one or more or a combination of the deviation angle (or a strabismus range), the deviation direction, and the fusion state information of the user and determine at least one or more or a combination of the deviation angle (or an deviation angle range), the deviation direction, and the fusion state information through the sensor.

The information of at least one or more or a combination of the deviation angle (or the deviation angle range), the deviation direction, and the fusion state information determined by the deviation angle determining unit 156 is stored in the storage unit 152.

The deviation angle determining unit 156 may include at least one or more cameras that capture the user's eye and may be configured to have an image analysis processing function to analyze images captured by the camera.

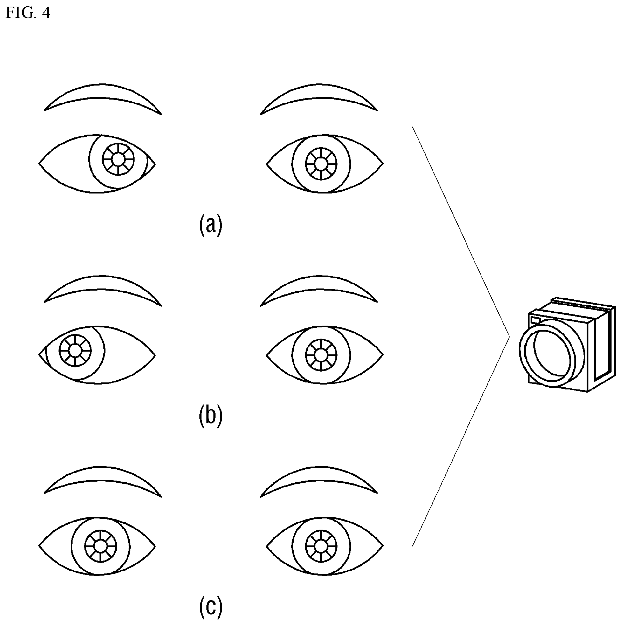

As shown in FIG. 4, the deviation angle determining unit 156 performs image analysis processing on videos captured by the camera to track the user's eye (for example, subtle changes in the pupil) and determine at least one or more or a combination of the deviation angle (or the deviation angle range), the deviation direction, and the fusion state information.

For example, in the SF interval, each time the prism diopter is changed, the gaze of the user with strabismus moves in a direction different from a direction intended by the change in prism diopter, causing the pupil to tremble, and after a certain period of time, the pupil adapts, and the trembling stops. When the prism diopter is changed again, the trembling occurs again, and after a certain period of time, the pupil adapts, and the trembling stops. In the FF interval, the gaze, i.e., the pupil, moves in the direction intended by the change in prism diopter without trembling. When the prism diopter is changed to the separation point after passing through the SF interval, the user's gaze does not follow the direction intended by the change in prism diopter, and the user's gaze shifts back to the original deviation direction. As the prism diopter is continuously changed from the separation point towards the deviation angle (i.e., if the prism diopter is increased), the user's gaze direction remains fixed and does not change from the deviation direction until a specific prism diopter is reached, from which the user's gaze direction begins to change to the gaze direction intended by the prism diopter, and this prism diopter can be detected as the recovery point. As the prism diopter is changed from the recovery point towards the deviation angle, the user's gaze moves in the direction intended by the change in prism diopter, causing the pupil to move. In this way, image analysis processing is performed on the videos captured by the camera to track the user's eye (for example, the subtle changes in the pupil) and determine at least one or more or a combination of the deviation angle (or the deviation angle range), the deviation direction, and the fusion state information.

Here, the "gaze" or "gaze direction" of the eye can be defined by an imaginary line created by light which is incident on the cornea and lens of the eye and reaches the fovea of the retina.

As described above, the deviation angle determining unit 156 performs image analysis processing on the videos captured by the camera, for example, to detect at least one or more or a combination of the deviation angle (or the deviation angle range), the deviation direction, and the fusion state information of the user's eye and determine the range within which the user's left eye, right eye, or both eyes can move (hereinafter referred to as "eye movement range"), and stores the eye movement range in the storage unit 152.

For example, the eye movement range stored in the storage unit 152 can be used to set the vision training range suitable for the user's eye. Particularly for users in infancy, it is preferable to perform vision training after detecting the maximum range within which the eye can move since the muscles around their eye may not be fully developed.

The eye movement range preferably includes the prism diopters (PD5 to PD19) from the prism diopter (PD5) corresponding to the start point of the FF interval to the prism diopter (PD19) (hereinafter referred to as a "final fusion prism diopter") corresponding to a last fusion point at which the final fusion state is provided just before the separation point (PD20) at which the double vision appears. More preferably, the eye movement range includes the prism diopters (PD10 to PD19) from the prism diopter (PD10) corresponding to the deviation angle to the final fusion prism diopter (PD19). Here, the eye movement range does not necessarily match the training range which will be described later. Preferably, the training range described later is within or the same as the eye movement range.

The control unit 151 includes a strabismus correction value determining unit 151B and a vision training unit 151C.

The strabismus correction value determining unit 151A determines a strabismus correction value used for conducting vision training to improve the user's strabismus based on the deviation angle and the deviation direction of the user determined by the deviation angle determining unit 156 or the ophthalmologist and stores the strabismus correction value in the storage unit 152. Here, the strabismus correction value refers to correction information used to move the user's eye in the opposite direction to the determined deviation angle to improve the user's strabismus and may include a correction direction and a correction prism diopter (or a correction refraction angle). The correction direction refers to a direction different from the deviation direction, preferably the exact opposite direction to the deviation direction. The correction prism diopter includes at least one or more prism diopter to move the eye as desired.

For example, when the user's eye is deviated with the deviation angle (PD10) of 16 PD (△) in the deviation direction of 30°, the prism diopter corresponding to the separation point (PD19) is 11 PD, and the final fusion prism diopter (PD19) is 11.5 PD, the deviation angle correction value includes the correction direction indicating 210° (see FIG. 18) and at least one or more or a combination of the correction prism diopters PD11 (15.5 PD), PD12 (15 PD), PD13 (14.5 PD), PD14 (14 PD), PD15 (13.5 PD), PD16 (13 PD), PD17 (12.5 PD), PD18 (12 PD), and PD19 (11.5 PD). If the user is in a corrected eye state, for example, by wearing prism glasses, the deviation angle (PD10) becomes 0 PD, the prism diopter corresponding to the separation point (PD20) is -5 PD, and the final fusion prism diopter (PD19) is -5.5 PD. The deviation angle correction value includes the correction direction indicating 210° (see FIG. 18) and at least one or more or a combination of the correction prism diopters -0.5 PD, -1.0 PD, -1.5 PD, -2.0 PD, -2.5 PD, -3.0 PD, -3.5 PD, -4.0 PD, and - 4.5 PD. Here, a negative sign (-) in PD indicates a correction direction opposite to the deviation direction of the user. FIG. 18 is a diagram describing the deviation direction. As described above, the deviation direction can be represented by an angle.

The strabismus correction value determining unit 151A preferably determines at least one or more prism diopters within the eye movement range as the correction prism diopter.

The strabismus correction value determining unit 151A stores the information of the correction direction and the correction prism diopter determined in the storage unit 152.

The storage unit 152 includes user information, such as the user's name, age, and gender, and at least one or more or a combination of deviation angle information, deviation direction information, fusion state information, and strabismus correction value information of the user.

The storage unit 152 also stores vision training information for each user. The vision training information includes information such as a training range, a training time, a vision training history, and a training speed determined based on the fusion adaptation time measured at each of multiple correction prism diopters for each user. The training speed is the speed of changing the prism diopter to each of the correction prism diopters included in the training range or the maintenance time of the prism diopter maintained at the correction prism diopter. The training speed is preferably determined based on the fusion adaptation time measured at each of the multiple correction prism diopters, and is preferably proportional to or the same as, for example, the fusion adaptation time.

The storage unit 152 may include at least one or more or a combination of flash memory type, hard disk type, solid state disk (SSD) type), silicon disk drive (SDD) type, multimedia card micro type, card type memory (for example, SD or XD memory), random access memory (RAM), static random access memory (SRAM), read-only memory (ROM), electrically erasable programmable read-only memory (EEPROM), programmable read-only memory (PROM), magnetic memory, magnetic disk, and optical disk.

The vision training unit 151B performs vision training to improve strabismus based on at least one or more or a combination of the user information, the deviation angle information, the deviation direction information, the fusion state information, the strabismus correction value, and the vision training information stored in the storage unit.

The vision training unit 151B determines the training range by determining at least one or more correction prism diopter to be included in the training range among from the correction prism diopters on the basis of the deviation angle information, the fusion state information, and the strabismus correction value. For example, the training range is determined based on the deviation angle (or the strabismus range), the separation point, and the recovery point determined by the deviation angle determining unit 156.

The training range preferably includes at least one or more prism diopters within the eye movement range. For example, the training range may include at least one or more correction prism diopter in the range from the deviation angle to the last fusion point, or at least one or more correction prism diopter in the range from the recovery point to the last fusion point. Additionally, the training range may include at least one or more correction prism diopter in the range from the SF start point to the last fusion point.

Instead of using the fusion adaptation time of each prism diopter measured by the deviation angle determining unit 156, the vision training unit 151B may newly select at least one or more prism diopters from a prism diopter range within the eye movement range as a new unit and measure the fusion adaptation time for each selected prism diopter, and the strabismus correction value determining unit 151A may determine at least one or more selected prism diopters as the correction prism diopter and set the correction prism diopter as the training range.

The vision training unit 151B performs vision training while causing the user's eye to move by changing the prism diopter at a speed corresponding to the fusion adaptation time of each prism diopter measured by the deviation angle determining unit 156, on the basis of the at least one or more correction prism diopters, which are determined by the strabismus correction value determining unit 151A and included in the training range, and the correction direction.

For example, referring to FIG. 20, when the interval from the recovery point (PD12) to the last fusion point (PD19) is determined as the training range, the vision training unit 151B changes the prism diopter on the basis of the training speed or the maintenance time, which is determined for each prism diopter according to the fusion adaptation time measured at each of the prism diopters PD12 to PD19. When the prism diopter is sequentially changed from PD12 to PD19, the prism diopter decreases from PD12 to PD16 at a relatively fast training speed and decreases from PD17 to PD19 at a relatively slow training speed. When the prism diopter is changed from PD19 to PD12, the prism diopter increases from PD19 to PD16 at a relatively slow training speed and increases from PD15 to PD12 at a relatively fast training speed. The operation of changing the prism diopter is repeatedly performed, so the user's gaze direction moves, within the eye movement range, in the deviation direction and then in the correction direction, thereby achieving training capable of strengthening the weak muscles that cause strabismus and fundamentally correcting strabismus.

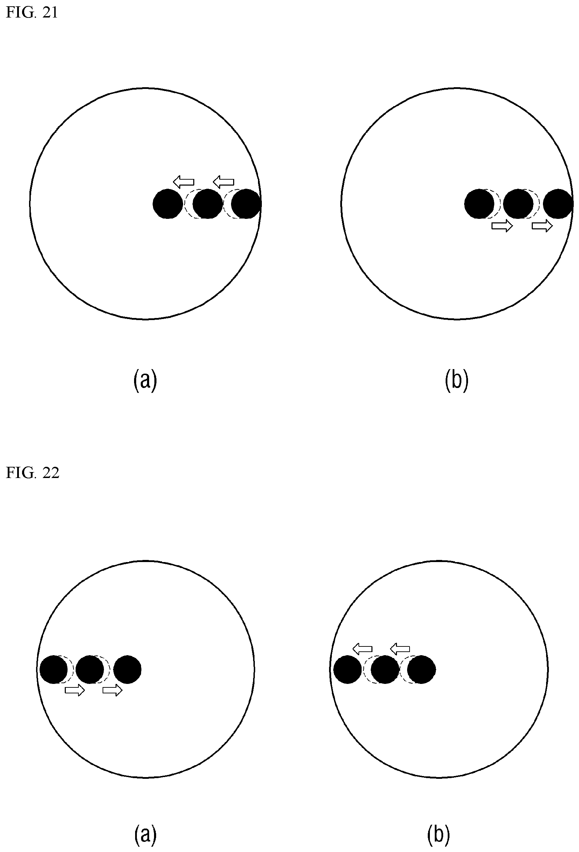

Referring to FIG. 21, in the case of exotropia, as the prism diopter is sequentially changed from PD12 to PD19, the gaze of the eye moves to the center (the correction direction) as shown in FIG. 21(a), and as the prism diopter is sequentially changed from PD19 to PD12, the gaze of the eye moves to the deviation direction as shown in FIG. 21(b). By reciprocating the eye in the deviation direction and the correction direction in this way, the weak muscles that cause strabismus can be strengthened, thereby improving the amount of deviation of the eye. Here, the gaze of the eye being at the center in the eye means the gaze direction of a normal person without strabismus when looking straight ahead.

Referring to FIG. 22, in the case of esotropia, as the prism diopter is sequentially changed from PD12 to PD19, the gaze of the eye moves toward the center as shown in FIG. 22(a), and as the prism diopter is sequentially changed from PD19 to PD12, the gaze of the eye moves to the deviation direction as shown in FIG. 22(b). By reciprocating the eye in the deviation direction and the correction direction in this way, the weak muscles that cause strabismus can be strengthened, thereby improving the amount of deviation of the eye.

Vision training is performed for hypertropia and hypotropia in a similar manner to esotropia and exotropia described above.

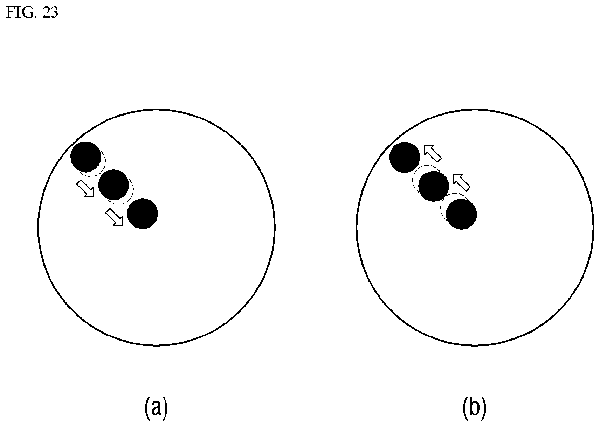

Referring to FIG. 23, even in the case of a specific deviation direction, as the prism diopter is sequentially changed from PD12 to PD19, the gaze of the eye moves toward the center as shown in FIG. 23(a), and as the prism diopter is sequentially changed from PD19 to PD12, the gaze of the eye moves to the deviation direction as shown in FIG. 23(b). By reciprocating the eye in the deviation direction and the correction direction in this way, the weak muscles that cause strabismus can be strengthened, thereby improving the amount of deviation of the eye.

The refraction angle adjusting unit 154 adjusts the refraction angle of the light incident on the user's eye and is configured to adjust the refraction angle of the light incident on the user's eye based on at least one or more or a combination of the deviation angle information, the deviation direction information, the fusion state information, and the vision training information, which are determined by the deviation angle determining unit 156 or the ophthalmologist.

The refraction angle adjusting unit 154 may include, for example, at least one or more prisms, at least one or more micromirrors, a liquid lens, at least one or more image processors, or a combination thereof, and a driving unit that drives them in order to adjust the refraction angle. If the refraction angle adjusting unit 154 is configured with at least one or more image processors, external images may be captured through a camera, and the refraction angle adjusting unit 154 may adjust an angle of projecting the captured external images on the user's eye based on at least one or more or a combination of the deviation angle information, deviation direction information, the fusion state information, and the vision training information.

However, the configuration of the refraction angle adjusting unit 154 according to an embodiment of the present invention is not limited to the configurations described above, and the refraction angle adjusting unit 154 may employ any configuration or element as long as it is possible to change or adjust the refraction angle of light incident on the user's eye.

For vision training, the refraction angle adjusting unit 154 obtains the training information from the storage unit 152 and changes or adjusts the refraction angle of light incident on the user's eye, for example, by changing the prism diopter according to the correction prism diopter, the training time, or the training speed included in the training information, thereby inducing the movement of the user's eye.

The user input unit 155 functions to receive information from the user, and when information is input through the user input unit 155, the control unit 151 can control the operation of the vision training device 100 based on the input information. The user input unit may include at least one or more hardware physical keys (hereinafter referred to as "hard keys") or software touch keys (hereinafter referred to as "soft keys") or a combination thereof. The hard keys may include, for example, buttons, dome switches, jog wheels, or jog switches, which are located on at least one of the front, back, and sides of the vision training device 100. The touch keys may include, for example, at least one or more or a combination of virtual keys or visual keys, which are displayed on a touchscreen type display unit through software processing, or touch keys which are located on parts other than the touchscreen. The virtual keys or the visual keys can have various forms and be displayed on the touchscreen, for example, as graphics, text, icons, videos, or combinations thereof.

Additionally, the user can input commands to perform specific operations through the user input unit 155. These commands can also be input through wired or wireless communication between the user terminal, such as a mobile phone, tablet, or PC, and the vision training device 100. For example, the user can input commands to turn on the power of the vision training device 100, turn off the power of the vision training device 100, start vision training, end vision training, search or select vision training modes, or adjust the refraction angle through the refraction angle adjusting unit 154.

The communication unit 152 can perform communication with the outside of the vision training device 100 through wired or wireless communication.

Hereinafter, a specific configuration example of the vision training device according to an embodiment of the present invention will be described in detail with reference to the accompanying drawings.

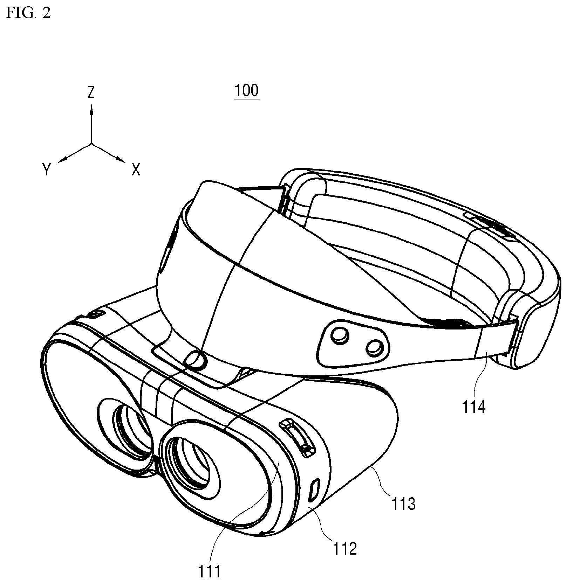

FIG. 2 is a perspective view illustrating the external appearance of the vision training device 100 according to the embodiment of the present invention. In FIG. 2, the vision training device 100 according to the embodiment of the present invention is illustrated as an integrated device implemented as a single device, but the vision training device 100 according to the present invention is not limited to such an integrated device, and each component thereof, which can be separated into different devices that implement the same role or operation, should be interpreted as falling within the scope of the present invention. For example, even when the refraction angle adjusting unit 154 is implemented as a separate configuration from the main body of the vision training device 100, this configuration should be interpreted as falling within the scope of the present invention as long as it is possible to perform vision training according to an embodiment of the present invention.

Referring to FIG. 2, the vision training device 100 according to an embodiment of the present invention may include a first housing 111, a second housing 112, a third housing 113, and a wearing part 114 as components constituting the external appearance of the present invention.

The first housing 111, the second housing 112, and the third housing 113 serve to protect the main body 120 described later from the outside.

The first housing 111 has at least one or more holes of a predetermined size at positions corresponding to at least one or more eyepiece holes 121a and 121b described later, so that light from the outside or a screen can enter the user's eye through the eyepiece holes 121a and 121b.

The second housing 112 may have holes of a predetermined size at positions corresponding to an adjustment lever 122a and 122b described later.

The third housing 113 has a part that comes into close contact with the area around the user's eyes, and the part that comes into close contact with the area around the user's eyes is made of a soft material with elasticity, such as sponge, so that the user does not feel discomfort while wearing the vision training device 100.

The wearing part 114 can be closely attached to the user's forehead and head, for example, so that the vision training device 100 does not come off from the user's worn state. The wearing part 114 can be connected to the second housing 112 or the third housing 113 through a connecting part such as a hinge.

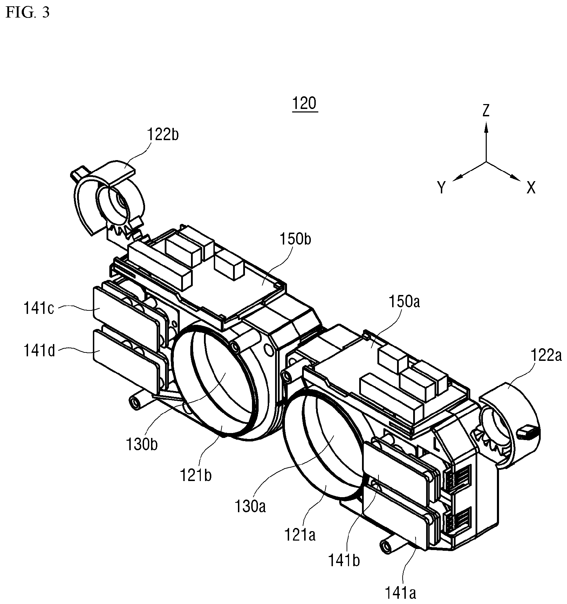

FIG. 3 is a perspective view showing an example of a main body 120 of the vision training device 100.

The main body 120 may include at least one or more eyepiece holes 121a and 121b, at least one or more adjustment levers 122a and 122b, at least one or more prism assemblies 130a and 130b, first motors 141a, 141b, 141c, and 141d of the driving unit 140, printed circuit boards 150a and 150b, and the user input unit 155 (see FIG. 1).

In the example of the vision training device 100 shown in FIG. 3, the refraction angle adjusting unit 154 shown in FIG. 1 is implemented by the prism assemblies 130a and 130b each having two prism lenses and the driving unit 140, but the refraction angle adjusting unit 154 of the present invention is not limited to the configuration including the prism assemblies 130a and 130b each having two prism lenses and the driving unit 140. For example, a configuration including at least one or more prism lens, three or more prism lenses, at least one or more micromirrors, at least one or more image processors, or at least one or more liquid lenses instead of the prism assemblies 130a and 130b each having two prism lenses also falls within the scope of the present invention, and the refraction angle adjusting unit 154 according to the present invention can have any configuration or element as long as it is possible to change the refraction angle of light incident on the user's eyes.

The main body 120 may include at least one or more eyepiece holes 121a and 121b which are formed at the positions corresponding to the user's eyes. The left eyepiece hole 121a is formed at a position corresponding to the user's left eye. The right eyepiece hole 121b is formed at a position corresponding to the user's right eye. Light from the outside or the screen can enter the user's eye through the left eyepiece hole 121a or the right eyepiece hole 121b.

The main body 120 may also include a left adjustment lever 122a and a right adjustment lever 122b. The user can adjust the distance between the prism lens assemblies 130a and 130b to match the distance between the user's eyes through the left adjustment lever 122a and the right adjustment lever 122b.

The main body 120 may include at least one of the left prism lens assembly 130a and the right prism lens assembly 130b described later. The left prism lens assembly 130a and the right prism lens assembly 130b each may be configured with at least one or more prism lenses. An embodiment of the present invention will be described focusing on the example where each of the prism assemblies 130a and 130b is configured with two prism lenses, but the scope of the present invention is not limited to this example.

The first motors 141a, 141b, 141c, and 141d of the driving unit 140 are configured to rotate multiple prism lenses 131a, 131b, 132a, and 132b to adjust or change the refraction angle of light incident on the prism lenses. For example, the first motor 141a rotates the left first prism lens 131a, the first motor 141b rotates the left second prism lens 132a, the first motor 141c rotates the right first prism lens 131b, and the first motor 141d rotates the right second prism lens 132b. A single first motor may be connected to transmit power to multiple prism lenses.

The vision training device 100 may include at least one or more printed circuit boards 150a and 150b on which a single chip or multiple chips are mounted to implement the operations of the control unit 151, the storage unit 152, the communication unit 153, the refraction angle adjusting unit 154, and the user input unit 155. The printed circuit board 150a may be provided on the left side of the main body 120, and the printed circuit board 150b may be provided on the right side of the main body 120.

The control unit 151 controls the driving unit 140. The control unit 151 can control the driving unit 140 to rotate at least one of the multiple prism lenses 131a, 131b, 132a, and 132b of the refraction angle adjusting unit 154. By rotating at least one of the multiple prism lenses 131a, 131b, 132a, and 132b, it is possible to change or adjust the refraction angle of the incident light to match the gaze direction of the user's left eye, right eye, or both eyes. The control unit 151 can also control the driving unit 140 to rotate at least one of the multiple prism lenses 131a, 131b, 132a, and 132b to change the refraction angle of the incident light according to the vision training information.

The control unit 151 can retrieve the vision training information of the user stored in the storage unit 152 and control the driving unit to rotate at least one of the multiple prism lenses according to the retrieved user vision training information.

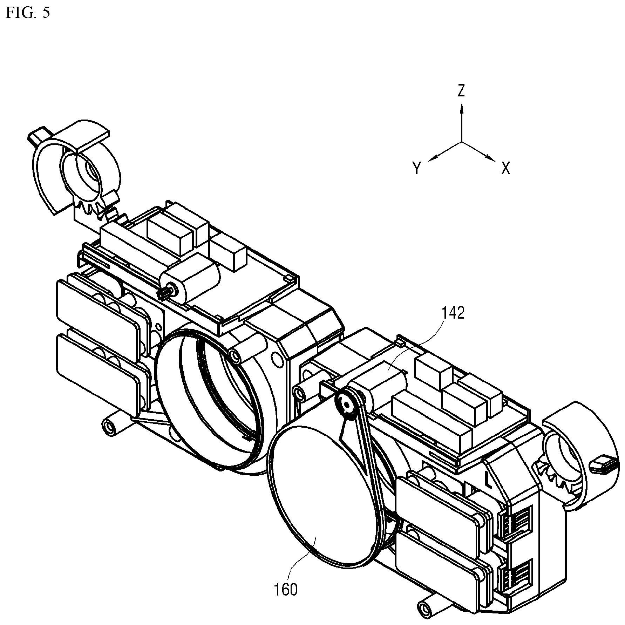

As shown in FIG. 5, the main body 120 may further include at least one or more blocking unit 160.

The blocking unit 160 may be disposed in front (+y direction) of the left eyepiece hole 121a or the right eyepiece hole 121b. The blocking unit 160 may be provided on both the left eyepiece hole 121a and the right eyepiece hole 121b.

The driving unit 140 may further include a second motor 142 for driving the blocking unit 160 to open and close. The control unit 151 can control the second motor 142 such that the blocking unit 160 blocks the light from being incident on the user's eye from at least one of the left eyepiece hole 121a or the right eyepiece hole 121b.

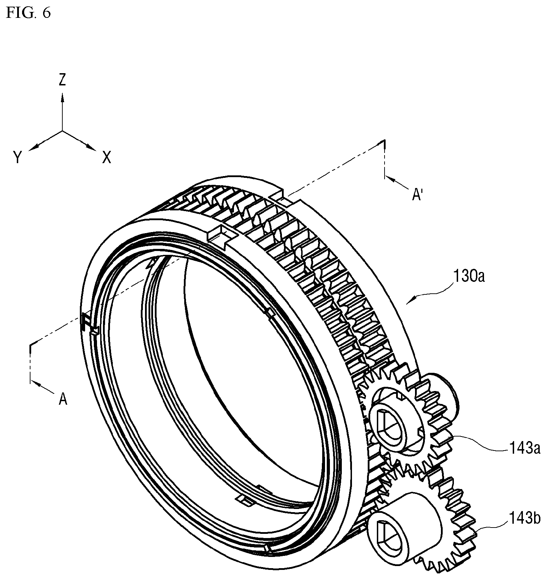

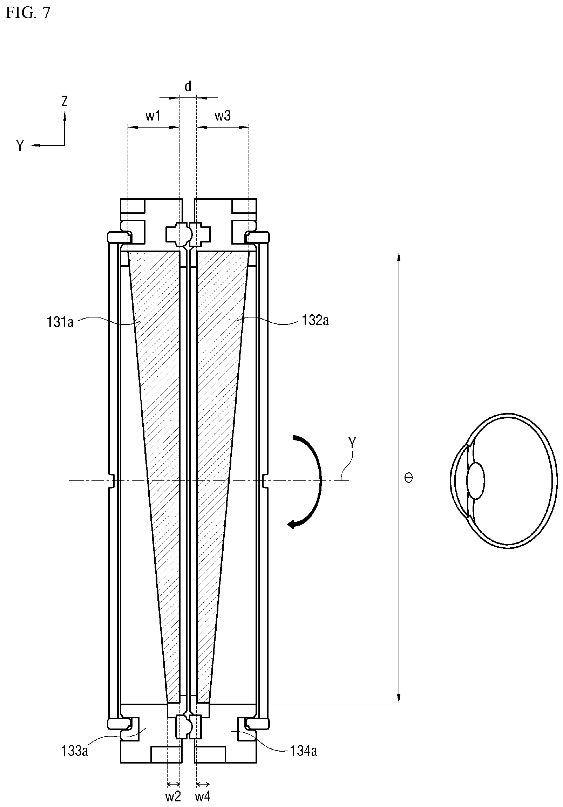

FIG. 6 is a perspective view of the prism lens assembly 130a and gears 143a and 143b according to an embodiment of the present invention, and FIG. 7 is a cross-sectional view of the prism lens assembly 130a taken along line A-A' of FIG. 5.

The prism lens assembly 130a is arranged in association with the left eye, and the prism lens assembly 130b is arranged in association with the right eye. As shown in FIG. 6, the left first prism lens 131a is arranged farther from the user's eye than the right second prism lens 132a. Similarly, the left first prism lens 131b is arranged farther from the user's eye than the right second prism lens 132b.

The first prism lens 131a is supported by a first prism lens support 133a, and the second prism lens 132a is supported by a second prism lens support 134a. Referring to FIG. 6, threads are formed on the first prism lens support 133a to engage with the rotating gear 143a, and threads are formed on the second prism lens support 132a to engage with the rotating gear 143b. For example, the driving force of the first motor 141a is transmitted to the first prism lens support 133a through the gear 143a, so the first prism lens 131a can be rotated independently of the second prism lens 132a. In a similar manner, the multiple prism lenses 131a, 131b, 132a, and 132b can be rotated independently.

The multiple prism lenses 131a, 131b, 132a, and 132b can rotate counterclockwise or clockwise around the rotation axis (Y). For example, the multiple prism lenses 131a, 131b, 132a, and 132b can rotate 360° counterclockwise and 360° clockwise.

Each of the multiple prism lenses 131a, 131b, 132a, and 132b can have a circular cross-section with a diameter (Φ). The first prism lens 131a can have a maximum thickness (w1) and a minimum thickness (w2) in the Y-axis direction, and the second prism lens 132a can have a maximum thickness (w3) and a minimum thickness (w4) in the Y-axis direction. The first prism lens 131a and the second prism lens 132a can be prism lenses of the same specification with the same diameter, maximum thickness, minimum thickness, and refractive deviation angle. Although the prism lenses with a circular cross-section is exemplarily described in an embodiment of the present invention, the scope of the present invention is not limited to this example, and prism lenses with triangular or rectangular cross-sections can also be employed.

The diameter (Φ) of the circular cross-section of the prism lens 131a or 132a can be, for example, 25 mm to 30 mm. The minimum thickness w2 or w4 of the prism lenses 131a or 132a is a thickness necessary to fix the lenses and is preferably 2 mm or less. The maximum thickness w1 or w3 of the prism lens 131a or 132a is preferably 2.3 mm or less.

Light from the outside passes through the first prism lens 131a and the second prism lens 132a and then enters the user's eyes. More specifically, the light is refracted according to refractive indices of first and second prism surfaces of the first prism lens 131a while passing through a first prism surface and a second prism surface of the first prism lens 131a in sequence, and then refracted again according to refractive indices of third and fourth prism surfaces of the second prism lens 132a while passing through the third prism surface and the fourth prism surface of the second prism lens 132a in sequence. A distance 'd' between the first prism lens 131a and the second prism lens 132a is defined as the distance between the second prism surface and the third prism surface. The distance 'd' is preferably designed to be as narrow as possible as long as the first prism lens 131a and the second prism lens 132a do not interfere with each other during their rotation. The first prism surface is inclined at a predetermined angle with respect to the second prism surface, and the fourth prism surface is inclined at a predetermined angle with respect to the third prism surface.

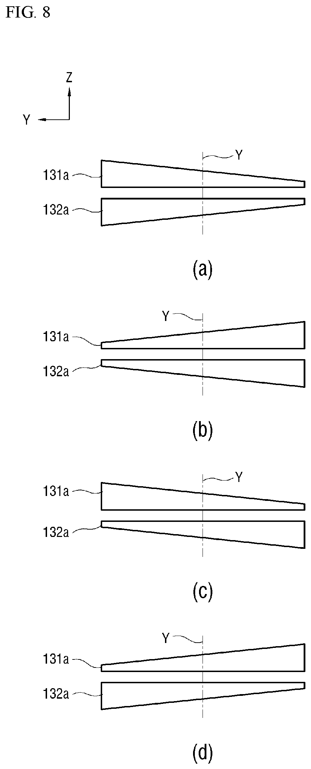

Each of the prism lenses 131a, 132a, 131b, and 132b has, for example, a prism diopter of 10△. When the first prism lens 131a having a prism diopter of 10△ and the second prism lens 132a having a prism diopter of 10△ are arranged as shown in FIG. 8(a) or FIG.8(b), the first prism lens assembly 130a having the prism lenses 131a and 132a can have a maximum prism diopter of 20△. When the bases of the first prism lens 131a and the second prism lens 132a are arranged exactly opposite to each other as shown in (c) or (d) of FIG. 8, that is, when the first prism lens assembly 130a does not rotate and the second prism lens 132a rotates 180° around the rotation axis (Y) from the state of FIG. 8(a) or FIG.8(b), the light passing through the first prism lens assembly 130a is not refracted as a result. In this case, the first prism lens assembly 130a has a prism diopter of 0△, which is defined as the first prism lens assembly 130a being in a neutral state.

FIGS. 8(a) to 8(d) are schematic diagrams of the prism lenses viewed from the X-Y plane. For convenience of description, a method of defining the positions of the prism lenses based on the first prism lens 131a and the second prism lens 132a will be described.

The positions of the prism lenses can be represented by listing a direction in which the part of the first prism lens 131a with the maximum thickness (base) is facing and a direction in which the part of the second prism lens 132a with the maximum thickness (base) is facing as an ordered pair. For example, the position of the prism lens in the case of FIG. 7(a) is represented as (+x, +x), the position of the prism lens in the case of FIG. 7(b) is represented as (-x, -x), the position of the prism lens in the case of FIG. 7(c) is represented as (+x, -x), and the position of the prism lens in the case of FIG. 7(d) is represented as (-x, +x). When the incident light and the refracted light passing through the prism lens assembly 130a are parallel to each other as shown in FIG. 7(c) and FIG. 7(d), the prism lens assembly 130a is in the neutral state.

As described above, each of the prism lenses 131a and 132a can freely rotate within a range of 360°. For example, each of the prism lenses 131a and 132a can rotate not only 0°, 45°, 90°, 135°, 180°, 225°, 270°, 315°, and 360° with respect to the reference axis but also at any angle between these angles. Therefore, it can be understood that there are countless positional relationships between the prism lenses in addition to the positional relationships shown in FIG. 8.

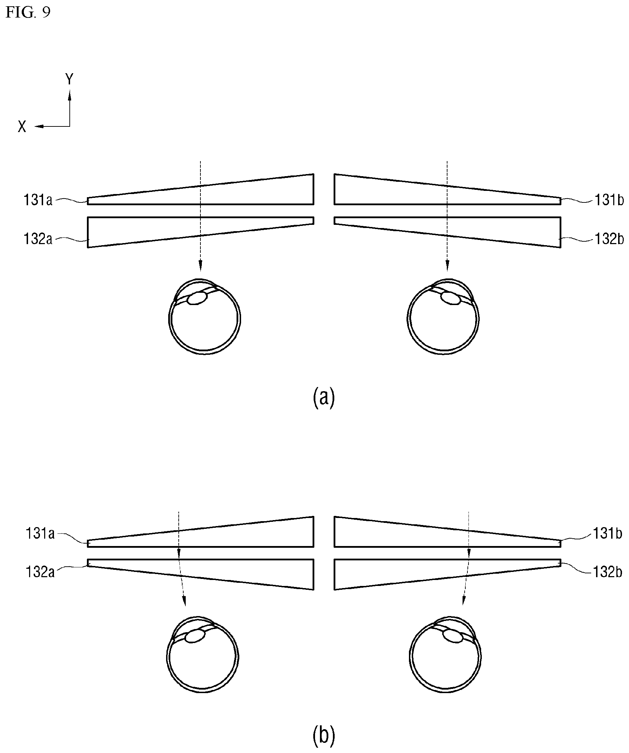

FIGS. 9(a) and 9(b) are views illustrating the user's eye with exotropia in an uncorrected state and the prism lenses 131a and 132a, which are viewed from the X-Y plane.

FIG. 9(a) shows a state where the gaze direction of the user's eye is turned outward at a predetermined angle (exotropia). FIG. 9(a) also shows that the direction of the light passing through the prism lenses 131b and 132b is not parallel to the gaze direction of the eye. Therefore, in the case of FIG. 9(a), it is difficult for the image to be accurately formed on the retina of the right eye, making it difficult to induce the user's vision training.

FIG. 9(b) shows the state where the direction of light passing through the prism lenses 131b and 132b is parallel to the gaze direction of the eye. Unlike the case in FIG. 9(a), in FIG. 9(b), the prism diopter provided by the prism lenses 131b and 132b is adjusted to the deviation angle or the strabismus range of the user, causing the image to be accurately formed on the retina. In the state in which the image is accurately formed on the retina, the multiple prism lenses can be controlled to rotate independently and freely to provide at least one or more desired prism diopters, guiding the movement of the eye within the eye movement range of the user in a manner similar to FIGS. 21 to 23 described later, thereby inducing the user's vision training.

In order to control the multiple prism lenses to rotate independently and freely to provide at least one or more desired prism diopters in the state in which the image accurately formed on the retina, only one of the two prism lenses may be rotated to provide a desired prism diopter, both of the prism lenses may be rotated symmetrically or asymmetrically (with different rotating amounts) in opposite directions to provide a desired prism diopter, or both of the prism lenses may be rotated in the same direction to provide a desired prism diopter.

This vision training can be performed with the user in a naked eye state (uncorrected eye state) or in a corrected eye state with separate prism glasses or eyeglass lenses.

FIG. 9(b) shows the case where both the prism assemblies 130b are in a base-in (BI) mode. Here, the prism assembly 130b being in the BI mode means that the first prism lens 131b and the second prism lens 132b are arranged so that the bases of the first prism lens 131b and the second prism lens 132b are both positioned inwards with respect to the user's eyes as shown in FIG. 8(b). That is, the left prism lens assembly 130a is in the (-x, +x) state, and the right prism lens assembly 130b is in the (+x, +x) state.

To change the prism assembly 130b in FIG. 9(a) from the neutral state to the BI mode in FIG. 8(b), the second prism lens 132b is rotated 180°.

Specifically, by rotating the right second prism lens 132b clockwise with the +Y direction as the rotation axis (Y) of the right second prism lens 132b, the prism assembly 130b in FIG. 9(a) can be shifted from the neutral state to the BI mode in FIG. 8(b). In other words, the second prism lens 132b can be rotated so that the part with the minimum thickness passes the upper side of the user's eye.

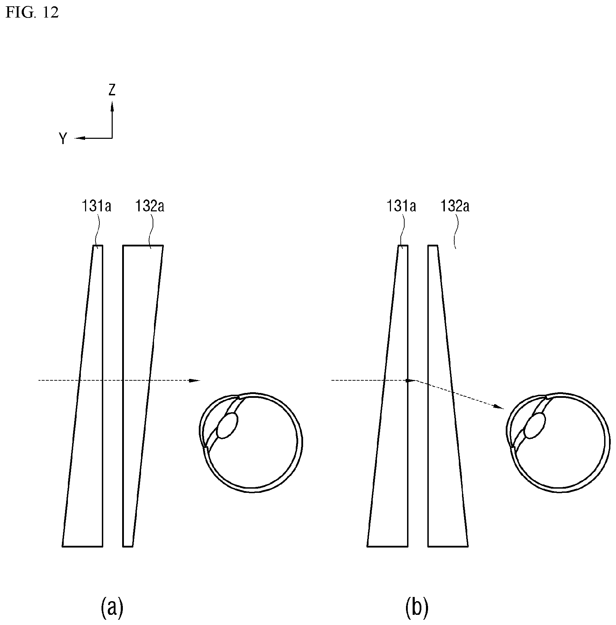

As described above, the prism assembly 130b in FIG. 9(a) can be shifted from the neutral state to the BI mode in FIG. 9(b) but can also be shifted from any neutral state to the BI mode in FIG. 9(b). Specifically, in order to perform control such that the left prism assembly 130a is shifted from the neutral state as in FIG. 12(a) to the BI mode, the left first prism lens 131a can be controlled to rotate 90° counterclockwise with respect to the gaze direction, and the left second prism lens 132a can be controlled to rotate 90° clockwise with respect to the gaze direction. As the left first prism lens 131a and the left second prism lens 132a are rotated in the opposite directions, the vertical refraction of the entire left prism assembly 130a is canceled out.

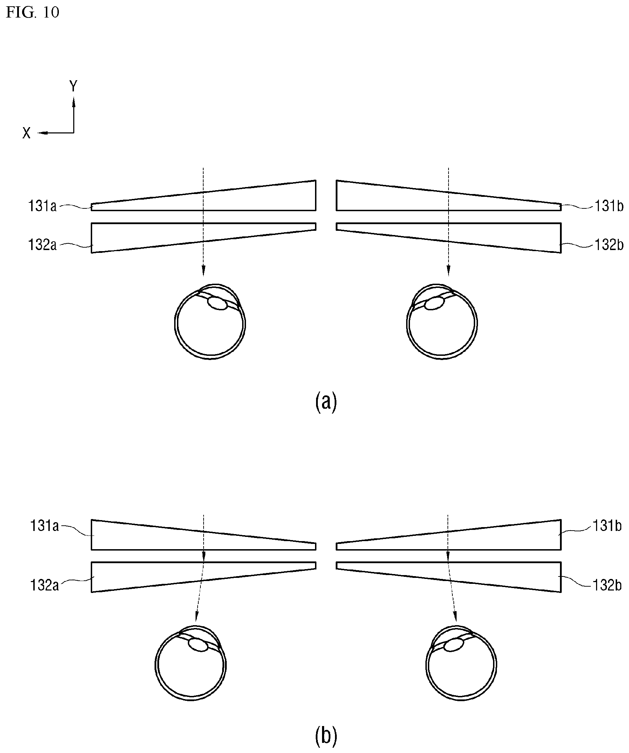

FIGS. 10(a) to 10(b) are views illustrating the user's eyes with esotropia and the multiple prism lenses, which are viewed on the X-Y plane.

FIGS. 10(a) and 10(b) both show the state where the gaze direction of the user's eye is turned inward at a predetermined angle (esotropia). FIG. 10(a) shows the state where the direction of light passing through the prism lens 131a is not parallel to the gaze direction of the eye, and FIG. 10(b) shows the state where the direction of light passing through the prism lenses 131a and 132a is parallel to the gaze direction of the eye.

For the same reasons as described above, in the case of FIG. 10(b), the prism diopter provided by the prism lenses 131a and 132a is adjusted to align with the deviation angle or the strabismus range of the user, causing the image to be accurately formed on the retina. In the state where the image is accurately formed on the retina, the multiple prism lenses can be controlled to rotate independently and freely to provide at least one or more desired prism diopters, guiding the movement of the eye within the eye movement range of the user in a manner similar to FIGS. 21 to 23 described later to thereby conduct vision training.

FIG. 10(b) shows the case where both the prism assemblies 130a are in a base-out (BO) mode. Here, the prism assembly 130a being in the BO mode means that the first prism lens 131a and the second prism lens 132a are arranged so that the bases of the first prism lens 131a and the second prism lens 132a are both positioned outwards with respect to the user's eyes as shown in FIG. 10(b). That is, the left prism lens assembly 130a is in the (+x, +x) state, and the right prism lens assembly 130b is in the (+x, -x) state.

To change the prism assembly 130a in FIG. 10(a) from the neutral state to the BO mode in FIG. 10(b), the left first prism lens 131a can be rotated 180°.

Specifically, by rotating the left first prism lens 131a clockwise with the +Y direction as the rotation axis (Y) of the left first prism lens 131a, the prism assembly 130a in FIG. 10(a) can be shifted from the neutral state to the BO mode in FIG. 10(b). In other words, the first prism lens 131a can be rotated so that the part with the minimum thickness thereof passes the upper side of the user's eye.

As described above, the prism assembly 130a in FIG. 10(a) can be shifted from the neutral state to the BO mode in FIG. 10(b) but can also be shifted from any neutral state to the BO mode in FIG. 10(b). Specifically, in order to perform control such that the left prism assembly 130a is shifted from the neutral state as in FIG. 12(a) to the BO mode in FIG. 10(b), the left first prism lens 13 1a can be controlled to rotate 90° clockwise with respect to the gaze direction, and the left second prism lens 132a can be controlled to rotate 90° counterclockwise with respect to the gaze direction. As the left first prism lens 131a and the left second prism lens 132a are rotated in the opposite directions, the vertical refraction of the entire left prism assembly 130a is canceled out.

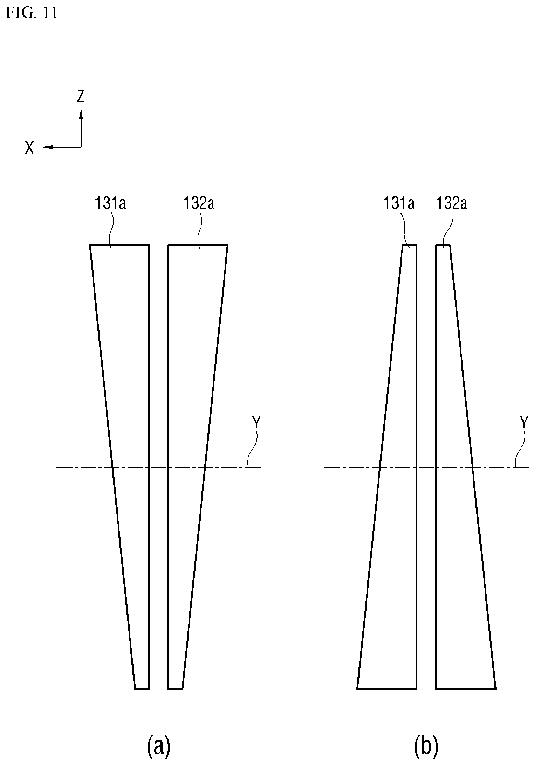

FIGS. 11(a) to 11(b) are schematic diagrams of the prism lenses, which are viewed on the Y-Z plane according to an embodiment of the present invention.

According to the method of defining the position of the prism lenses described above, in the case of FIG. 11(a), the left prism lens assembly 130a is in the (+z, +z) state, and in the case of FIG. 10(b), it is in the (-z, -z) state.

Since the prism lenses 131a and 132a can each rotate 360° counterclockwise or clockwise as described above, it can be understood that there are countless positional relationships between the prism lenses in addition to the positional relationships shown in FIG. 10.

FIGS. 12(a) and 12(b) are views illustrating the user's eye with hypertropia and the multiple prism lenses, which are viewed on the Y-Z plane.

FIGS. 12(a) and 12(b) show the state where the gaze direction of both eyes of the user is turned upward at a predetermined angle (hypertropia). FIG. 12(a) shows the state where the direction of light passing through the prism lenses 131a and 132a is not parallel to the gaze direction of the eye, and FIG. 12(b) shows the state where the direction of light passing through the prism lenses 131a and 132a is parallel to the gaze direction of the eye.

For the same reasons as described above, in the case of FIG. 12(b), the prism diopter provided by the prism lenses 131a and 132a is adjusted to align with the deviation angle or the strabismus range of the user, causing the image to be accurately formed on the retina. In the state where the image is accurately formed on the retina, the multiple prism lenses can be controlled to rotate independently and freely to provide at least one or more desired prism diopters, guiding the movement of the eye within the eye movement range of the user in a manner similar to FIGS. 21 to 23 described later to thereby conduct vision training.

FIG. 12(b) shows the case where the prism assembly 130a is in a base-down (BD) mode. Here, the prism assembly 130a being in the BD mode means that the first prism lens 131a and the second prism lens 132a are arranged so that the thickness thereof increases downward with respect to the user's eye as shown in FIG. 12(b). That is, the left prism lens assembly 130a is in the (-z, -z) state.

To change the prism lens assembly 130a from the neutral state in FIG. 12(a) to the BD mode in FIG. 12(b), the second prism lens 132a can be rotated 180°.

Specifically, in order to change from the neutral state in FIG. 9(a) to the BD mode in FIG. 12(b) or from the neutral state in FIG. 10(a) to the BD mode in FIG. 12(b), the neutral state as in FIG. 12(a) can be created first, and then the neutral state is shifted to the BD mode in FIG. 12(b).

Specifically, the first prism lens 131a and the second prism lens 132a are controlled to simultaneously rotate 90° in the same direction around the Y-axis to create the neutral state as in FIG. 12(a) first, and then only the second prism lens 132a is rotated around the Y-axis to thereby create the BD mode in FIG. 12(b). Alternatively, by controlling the first prism lens 131a and the second prism lens 132a to rotate in the different directions around the Y-axis, the BD mode in FIG. 12(b) can be created.

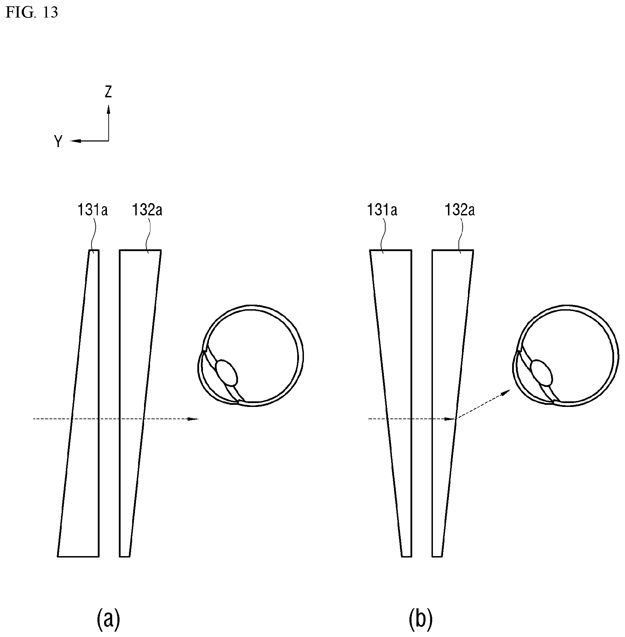

FIGS. 13(a) and 13(b) are views illustrating the user's eye with hypotropia and the multiple prism lenses, which are viewed from the Y-Z plane.

FIGS. 13(a) and 13(b) show the state where the gaze direction of both eyes of the user is turned downward at a predetermined angle (hypotropia). FIG. 13(a) shows the state where the direction of light passing through the prism lenses 131a and 132a is not parallel to the gaze direction of the eye, and FIG. 13(b) shows the state where the direction of light passing through the prism lenses 131a and 132a is parallel to the gaze direction of the eye.

For the same reasons as described above, in the case of FIG. 13(b), the prism diopter provided by the prism lenses 131a and 132a is adjusted to align with the deviation angle or the strabismus range of the user, causing the image to be accurately formed on the retina of the left eye. In the state where the image is accurately formed on the retina, the multiple prism lenses can be controlled to rotate independently and freely to provide at least one or more desired prism diopters, guiding the movement of the eye within the eye movement range of the user in a manner similar to FIGS. 21 to 23 described later to thereby conduct vision training.

FIG. 13(b) shows the case where the prism assembly 130a is in a base-up (BU) mode. Here, the prism assembly 130a being in the BU mode means that the first prism lens 131a and the second prism lens 132a are arranged so that the thickness thereof increases upward with respect to the user's eye as shown in FIG. 13(b). That is, the left prism lens assembly 130a is in the (+z, +z) state.

To change the left prism lens assembly 130a from the neutral state in FIG. 13(a) to the BU mode in FIG. 13(b), the first prism lens 131a can be rotated 180°.

Specifically, in order to change from the neutral state in FIG. 9(a) to the BU mode in FIG. 13(b) or from the neutral state in FIG. 10(a) to the BU mode in FIG. 13(b), the neutral state as in FIG. 13(a) can be created first, and then the neutral state transitions to the BU mode in FIG. 13(b).

Specifically, the first prism lens 131a and the second prism lens 132a are controlled to simultaneously rotate 90° in the same direction to create the neutral state in FIG. 13(a) first, and then only the first prism lens 131a is rotated to thereby create the BU mode in FIG. 13(b). Alternatively, by controlling the first prism lens 131a and the second prism lens 132a to rotate in the different directions around the Y-axis, the BU mode in FIG. 13(b) can be created.

As described above, the prism assembly 130a can be shifted from the neutral state in FIG. 13(a) to the BU mode in FIG. 13(b) but can also be shifted from any neutral state to the BU mode in FIG. 13(b).

In this specification, the esotropia in FIG. 19(a), the exotropia in FIG. 19(b), the hypertropia in FIG. 19(c), and the hypotropia in FIG. 19(d) are described as the representative examples of strabismus states, but the prism lenses 131a, 132a, 131b, and 132b of the vision training device 100 can freely rotate with a desired angle in either counterclockwise or clockwise direction, leading to the effect of enhancing or improving binocular fusion force for strabismus in any direction other than esotropia, exotropia, hypertropia, and hypotropia.

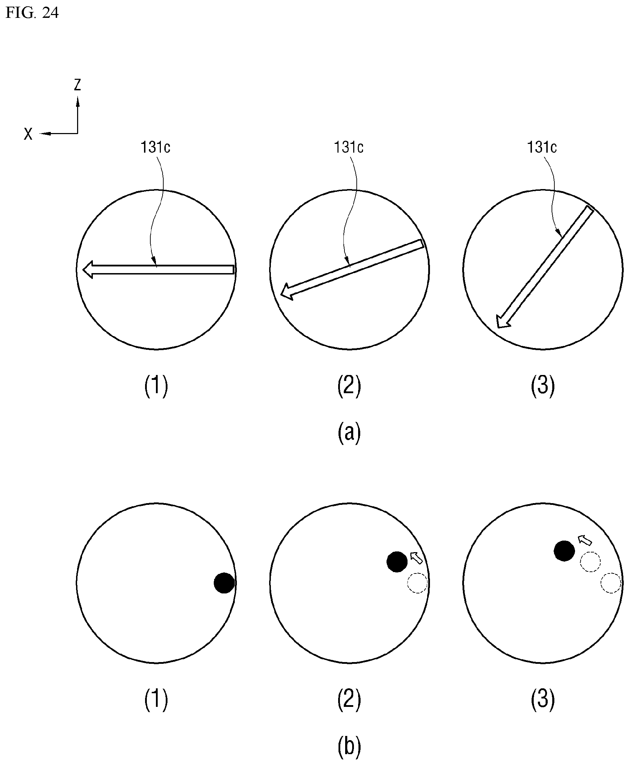

FIG. 24 is a diagram showing the refraction angle determining unit 154 including a single prism lens. In FIG. 24, 131c represents a prism lens, and a part corresponding to the head of an arrow represents the base. In FIG. 24(a), the prism lens is in the BI mode, and

FIG. 24(b) shows the case where the user has exotropia. The prism lens 131c has a prism diopter associated with the deviation angle of the user to improve the user's strabismus. When the vision training unit 151B conducts vision training, the refraction angle adjusting unit 154 changes the refraction angle by changing its prism diopter to at least one or more correction prism diopters included in the training range on the basis of the deviation angle information, the strabismus correction value information, the deviation direction information, and the vision training information included in the vision training information in the order of (1), (2), (3) in FIG. 24(a). Accordingly, the user's gaze moves in the order of (1), (2), (3) as shown in FIG. 24(b).

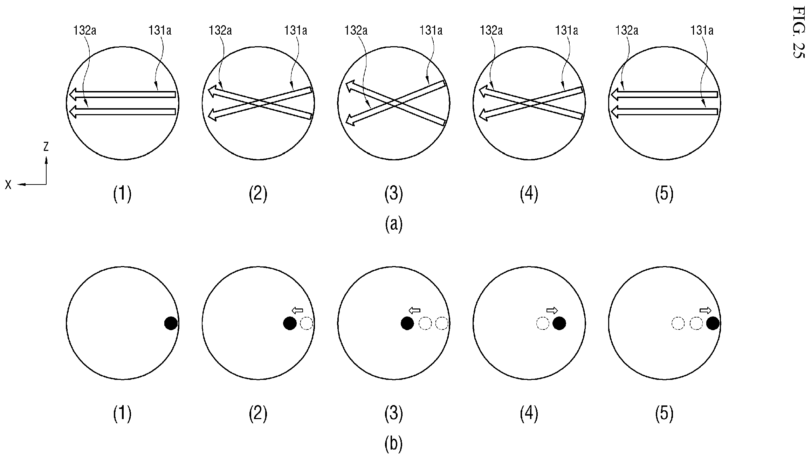

However, when using a single prism, the user's gaze moves in an elliptical manner as shown in FIG. 25(b), rather than moving in a substantially linear manner as shown in FIGS. 20 to 23. Especially when the user's gaze moves in an elliptical manner in the upward direction, the user's eye fatigue increases, causing dizziness.

To prevent the user's gaze from moving in an elliptical manner in the upward direction during vision training, it is preferable to control the prism lens 131a, 131b, 132a, and 132b included in the prism assemblies 130a and 130b to rotate symmetrically in the different directions (with the same amount of rotation for each prism lens) or to rotate asymmetrically (with different amounts of rotation for each prism lens) while causing the gaze to move in an elliptical manner in the downward direction. For this purpose, as described above, the prism lens 131a, 131b, 132a, and 132b included in the prism assemblies 130a and 130b are configured to rotate independently.

FIG. 25 is a schematic diagram showing the vision training of the vision training device 100 including the refraction angle determining unit 154 including two prism lenses according to an embodiment of the present invention. In FIG. 25, 131a and 132a represent prism lenses, and the part corresponding to the head of the arrow represents the base. In FIG. 25, each of the two prism lenses is in the BI mode, and vision training is conducted in an uncorrected eye state where the user is not wearing the prism lenses glasses, and the user has exotropia. The prism diopter provided by the two prism lenses 131a and 131b has a prism diopter associated with the deviation angle of the user to improve the user's strabismus. When the vision training unit 151B conducts the vision training, the refraction angle adjusting unit 154 changes the refraction angle, preferably, while controlling the two prism lenses 131a and 131b) to rotate in the different directions such that its prism diopter is changed to at least one or more correction prism diopters included in the training range on the basis of at least one or a combination of the vision training information and the deviation angle information, the strabismus correction value information, the deviation direction information, and the fusion state information, which are included in the vision training information, in the order of (1), (2), (3), (4), and (5) in FIG. 25(a). Accordingly, the user's gaze moves in a substantially linear manner in the order of (1), (2), (3), (4), and (5) as shown in FIG. 25(b). In this case, since the user's gaze moves in a substantially linear manner, it is possible to prevent eye fatigue or dizziness from increasing.

The prism diopters associated with (2) to (4) in FIG. 25(a) and (b) are smaller than the deviation angle (PD12 in FIG. 20) and larger than the separation point (PD20 in FIG. 20). By repeatedly performing the operations of (1) to (5) in FIG. 25(a) and (b), the weak muscles that are the root cause of exotropia can be strengthened.

In the above embodiment, the refraction angle determining unit 154 including a single prism lens or two prism lenses is mainly described, but the refraction angle determining unit 154 can include three or more prism lenses. In this case, any configuration is possible as long as it is possible to control the prism lenses to rotate in independent directions, and the total refraction angle of the three or more prism lenses can be controlled to align with the deviation angle and the strabismus correction value.

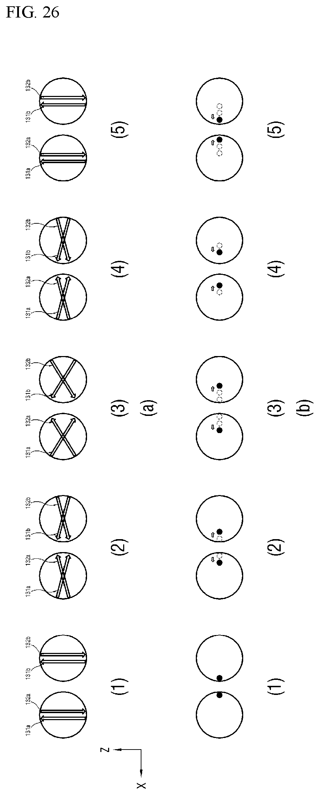

FIG. 26 is a schematic diagram showing the vision training of the vision training device 100 when both eyes have esotropia according to an embodiment of the present invention. FIG. 26 shows vision training to correct esotropia while the user is wearing prism glasses. In FIG. 26, 131a, 132a, 131b, and 132b represent prism lenses, and the part corresponding to the head of the arrow represents the base. The user's both eyes have substantially the same deviation angle, and the prism diopter provided by the two prism assemblies (131a, 132a, 131b, and 132b) has a prism diopter associated with the deviation angle of the user to improve the user's strabismus. When the vision training unit 151B conducts vision training, the two prism assemblies (131a, 132a, 131b, and 132b) included in the refraction angle adjusting unit 154 change the refraction angle, preferably, while controlling each pair of prism lenses (131a and 132a and 13 1b and 132b) to rotate in the different directions to correct different angles of deviation such that the prism diopter is changed to at least one or more correction prism diopters included in the training range on the basis of at least one or a combination of the vision training information and the deviation angle information, the strabismus correction value information, the deviation direction information, and the fusion state information, which are included in the vision training information or the vision training information, in the order of (1), (2), (3), (4), and (5) in FIG. 26(a). Accordingly, the user's gaze moves in a substantially linear manner in the order of (1), (2), (3), (4), and (5) as shown in FIG. 26(b).

The prism diopters corresponding to (2) to (4) in FIG. 26(a) and (b) are smaller than the deviation angle (PD12 in FIG. 20) and larger than the separation point (PD20 in FIG. 20). By repeatedly performing the operations of (1) to (5) in FIG. 26(a) and (b), the weak muscles that are the root cause of esotropia can be strengthened.

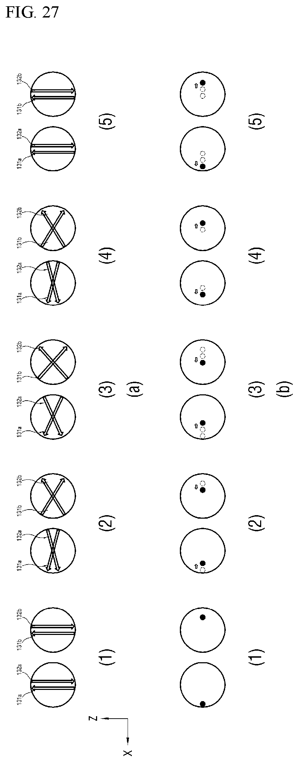

FIG. 27 is a schematic diagram showing the vision training of the vision training device 100 when both eyes have exotropia and the right eye and the left eye differ in the degree of strabismus, i.e., the deviation angle according to an embodiment of the present invention. FIG. 26 shows vision training to correct esotropia while the user is wearing prism glasses. In FIG. 26, for example, it is assumed that the deviation angle of the left eye is 10 PD and the deviation angle of the right eye is 2 PD. In FIG. 26, 131a, 132a, 131b, and 132b represent prism lenses, and the part corresponding to the head of the arrow represents the base. The prism diopter provided by the two prism assemblies (131a, 132a, 131b, and 132b) has a prism diopter associated with the deviation angle of the user to improve the user's strabismus. When the vision training unit 151B conducts vision training, the two prism assemblies (131a, 132a, 131b, and 132b) included in the refraction angle adjusting unit 154 change the refraction angle, preferably, while controlling each pair of prism lenses (131a and 132a and 131b and 132b) to rotate in the different directions such that the prism diopter is changed to at least one or more correction prism diopters included in the training range on the basis of at least one or a combination of the vision training information and the deviation angle information, the strabismus correction value information, the deviation direction information, and the fusion state information, which are included in the vision training information or the vision training information, in the order of (1), (2), (3), (4), and (5) in FIG. 27(a). Accordingly, the user's gaze moves in a substantially linear manner in the order of (1), (2), (3), (4), and (5) as shown in FIG. 27(b). Since both eyes have different angles of deviation, the difference in the amount of rotation of one pair of two prism lenses rotating in the opposite directions, which are included in the prism assemblies, differ from the difference in the amount of rotation of the other pair of two prism lenses rotating in opposite directions, which are included in the prism assemblies as shown in (2) to (4) of FIG. 27(a) and (b). That is, the difference in the amount of rotation of the two prism lenses 131a and 132a rotating in the opposite directions is different from the difference in the amount of rotation of the two prism lenses 131b and 132b rotating in the opposite directions.

The prism diopters corresponding to (2) to (4) in FIG. 27(a) and (b) are smaller than the deviation angle (PD12 in FIG. 20) and larger than the separation point (PD20 in FIG. 20). By repeatedly performing the operations of (1) to (5) in FIG. 27(a) and (b), the weak muscles that are the root cause of exotropia with different degrees in both eyes can be strengthened.

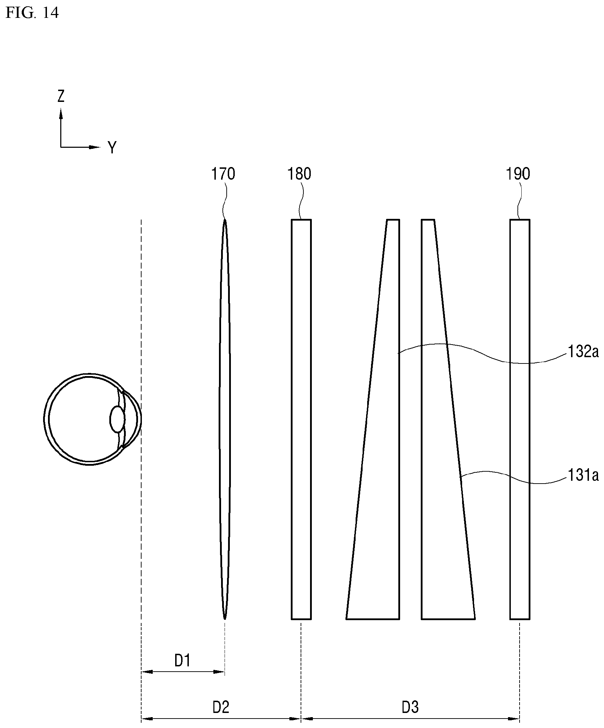

FIG. 14 is a diagram showing the vision training device in a corrected eye state in which the user wears prism glasses according to an embodiment of the present invention.

Referring to FIG. 14, the vision training device 100 according to an embodiment of the present invention includes a receiving part (not shown) that can accommodate prism glasses (170), which is designed according to the user's eye condition (vision, degree of strabismus, etc.) and required design. The vision training device 100 according to an embodiment of the present invention includes protective lenses 180 and 190 before and after the prism lenses 131a and 132a included in the refraction angle adjusting unit 154 to protect the prism lenses 131a and 132a from external impacts or dust.

As shown in FIG. 14, the arrangement can be in the order of the eye, the prism glasses 170, the protective lens 180, the prism lenses 131a and 132a, and the protective lens 190. A distance D1 between the eye and the apex of the prism glasses 170 can be, for example, 13mm. A distance D2 between the eye and the protective lens 180 can be, for example, 15mm to 20mm. A distance D3 between the protective lens 180 and the protective lens 190 is preferably designed to be as narrow as possible since the field of view is wider as the distance D3 decreases.

The vision training device 100 according to an embodiment of the present invention enables the user to conduct vision training in both corrected and uncorrected eye states as described above.

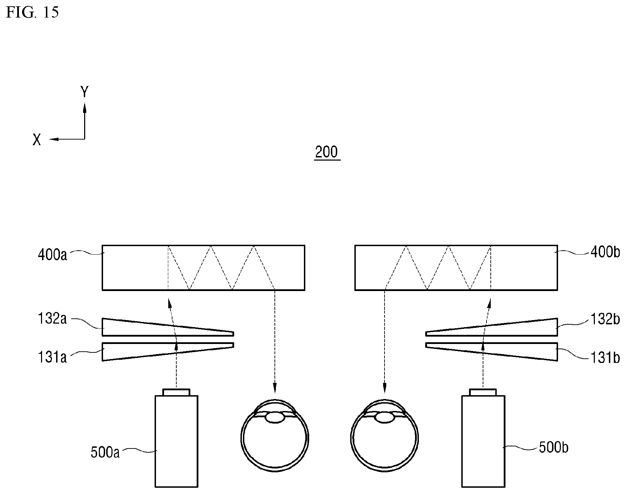

FIG. 15 is a diagram for describing a vision training device 200 with an augmented reality (AR) function according to another embodiment of the present invention. As shown in FIG. 15, the vision training device 200 may include image source units 500a and 500b that output augmented reality images and reflecting units 400a and 400b. Describing the left eye side, beams output from the image source unit 500a can pass through the left first prism lens 131a and the left second prism lens 132a and then enter the reflecting unit 400a. The beams entering the reflecting unit 400a can be reflected within the reflecting unit 400a and then enter the user's left eye. The same method can be applied to the right eye side. The control unit 151 controls the prism assemblies (131a, 132a, 131b, and 132b) to change the refraction angle of the light entering the user's eye so that the light entering the user's eye is parallel to the gaze direction of the user's eye, and changes the refraction angle of the light according to the vision training information during vision training to guide the movement of the user's eye. Specific operations and vision training method of the vision training device 200 according to another embodiment of the present invention shown in FIG. 15 are substantially the same as the specific operations and vision training method of the vision training device 100 according to the embodiment of the present invention, so description thereof are omitted.

As a modification of the vision training device 200 according to another embodiment of the present invention shown in FIG. 15, the vision training device 200 may be configured to employ micromirrors instead of the prism lenses 131a, 132a, 131b, and 132b. In this case, at least one or more micromirrors are disposed in the reflecting units 400a and 400b, and the control unit controls the micromirrors, on the basis of at least one or more or a combination of the deviation angle information, the deviation direction information, the strabismus correction value, and the vision training information, to control the refraction angle of the light entering the user's eye such that operations and vision training, which are similar to the vision training device 100 according to the embodiment of the present invention, are performed to guide the movement of the user's eye.

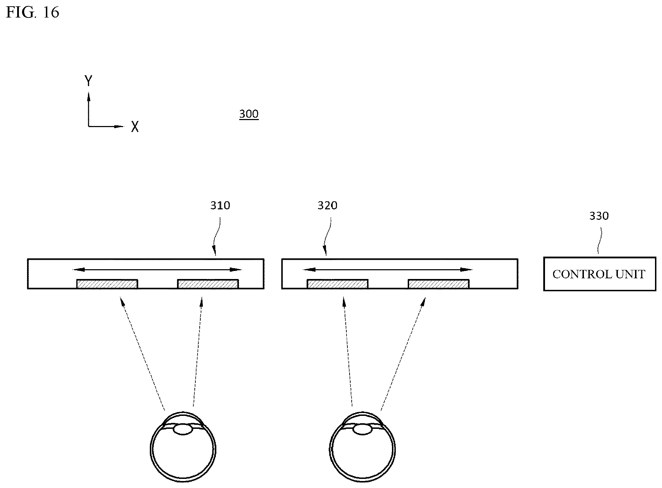

FIG. 16 is a diagram for describing a vision training device 300 with a virtual reality (VR) function according to another embodiment of the present invention. As shown in FIG. 16, display units 310 and 320 corresponding to both eyes are configured to move positions of displayed images under the control of a control unit 330, thereby guiding the movement of the user's eye during vision training.

A storage unit (not shown) stores the fusion state information such as the SF interval, the FF interval, the deviation angle (or the strabismus range), the recovery point, and the separation point, the deviation direction, the strabismus correction value, the training range, the eye movement range, and the like in association with coordinates of pixels.

The control unit 330 controls the display units 310 and 320 to display images at the coordinates of the pixels associated with the deviation angle of the user, the coordinates of the pixels associated with at least one or more correction prism diopters, or the like on the basis of the fusion adaptation time to thereby guide the movement of the user's eye. The control unit 330 causes the images to be displayed at the coordinates of the associated pixels on the basis of at least one or more or a combination of the deviation angle information, the deviation direction information, the strabismus correction value, and the vision training degree to thereby guide the movement of the user's eyes during vision training. The vision training device 300 with the VR function according to another embodiment of the present invention performs operations and vision training methods similar to the vision training device 100 according to the embodiment of the present invention.

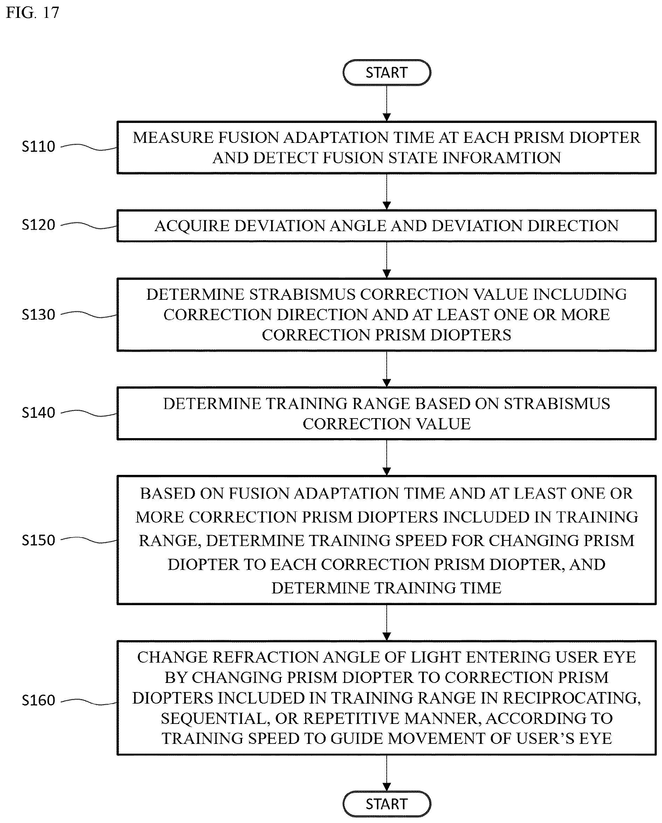

Next, a vision training method according to an embodiment of the present invention will be described with reference to FIG. 17. FIG. 17 is a flowchart showing the vision training method according to an embodiment of the present invention.

First, while changing the prism diopter with intervals of predetermined units (for example, 0.5 PD units or 1 PD units), the deviation angle determining unit 156 measures the fusion adaptation time at each prism diopter and detects the fusion state information of the user's eye (S110).
The fusion state information includes at least one or more or a combination of the SF start point, the separation point, the recovery point, the SF interval, and the FF interval of the eye.

Then, the deviation angle information and the deviation direction information are acquired through the deviation angle determining unit 156 (S120). The strabismus correction value including the correction direction and at least one or more correction prism diopters is determined on the basis of the deviation angle information and the deviation direction information (S130).

Then, based on the fusion state information and the strabismus correction value, the training range including at least one or more correction prism diopters is determined (S140), and based on the fusion adaptation time at least one or more correction prism diopters included in the training range, the speed (training speed) or time for changing the prism diopter to each correction prism diopter is determined, and the training time is determined (S150).

Then, by changing the prism diopter to at least one or more correction prism diopters included in the training range in a reciprocating, sequential, or repetitive manner, according to the training speed, the refraction angle of the light entering the user's eye is changed through the refraction angle adjusting unit 154 to guide the user's eye to move in a direction (preferably the opposite direction) different from the deviation direction, thereby strengthening the weak eye muscles causing strabismus (S160).

Meanwhile, the disclosed vision training method can be implemented in the form of a recording medium having computer-executable instructions stored therein. The instructions can be stored in the form of program codes, and when executed by a processor, can generate program modules to perform the operations of the disclosed embodiments. The recording medium can be implemented as a computer-readable recording medium.

The computer-readable recording medium includes all types of recording media storing instructions that can be decoded by a computer. Examples of the computer-readable recording medium include read only memory (ROM), random access memory (RAM), magnetic tape, magnetic disk, flash memory, and optical data storage devices.

As described above, the disclosed embodiments have been described with reference to the accompanying drawings. Those skilled in the art to which the present invention pertains will understand that the present invention can be implemented in other forms without changing the technical spirit or essential features of the present invention. The disclosed embodiments are illustrative and should not be construed as limiting.

## Claims

1. A vision training device, comprising:
a storage unit that stores a deviation angle of a user; and
a control unit that guides movement of the user's eye based on the deviation angle.

2. The vision training device according to claim 1, wherein the control unit comprises
a strabismus correction value determining unit that determines a strabismus correction value based on the deviation angle, and
a vision training unit that sets a training range based on the strabismus correction value and guides the movement of the user's eye based on the training range.

3. The vision training device according to claim 2, wherein:
the storage unit stores information indicating a deviation direction,
the strabismus correction value includes a correction direction opposite to the deviation direction and at least one or more correction prism diopters smaller than the deviation angle, and
the training range includes at least one or more of the at least one or more correction prism diopters.

4. The vision training device according to claim 3, wherein the training range includes an interval where fusion is performed relatively quickly and an interval where fusion is performed relatively slowly.

5. A vision training device, comprising:
a storage unit that stores information indicating deviation of the user's eye and at least one or more pieces of correction information for correcting the deviation of the user's eye; and
a control unit that guides movement of the user's eye in a direction different from a direction of the deviation based on at least one of the at least one or more pieces of correction information.

6. The vision training device according to claim 5, wherein the at least one or more pieces of correction information differ in time taken for fusion of both eyes.

7. The vision training device according to claim 6, wherein the at least one or more pieces of correction information guides the user's eye to move in a direction opposite to the direction of the deviation.

8. The vision training device according to claim 7, wherein the at least one or more pieces of correction information are at least one or more prism diopters.

9. The vision training device according to claim 8, wherein the at least one or more prism diopters includes at least one or more or a combination of a prism diopter associated with a deviation angle of the user's eye, a prism diopter which is smaller than the deviation angle and smallest to achieve fusion of the user's eye, and a prism diopter which is smaller than the deviation angle and larger than the smallest prism diopter.

10. The vision training device according to claim 9, wherein the prism diopter which is smaller than the deviation angle and larger than the smallest prism diopter is a prism diopter at which double vision becomes single vision.

11. A vision training method, comprising:
measuring a fusion adaptation time for each of a plurality of refraction angles smaller than a deviation angle of a user's eye;
determining a training time for changing the refraction angle to the plurality of refraction angles based on the fusion adaptation time;
determining at least one or more of the plurality of refraction angles as a training range; and
changing the refraction angle to the at least one or more refraction angles included in the training range according to the training time to guide movement of the user's eye.

12. The vision training method according to claim 11, wherein the at least one or more refraction angles included in the training range include a refraction angle at which double vision becomes single vision.

13. The vision training method according to claim 12, wherein the at least one or more refraction angles included in the training range include the smallest refraction angle at which both eyes achieve fusion.

14. The vision training method according to claim 13, wherein the at least one or more refraction angles included in the training range include a deviation angle of the user's eye.

15. The vision training method according to claim 14, wherein the fusion adaptation times taken to achieve function of both eyes, which are measured for the plurality of refraction angles, are different from each other.
